# EUROPEAN PATENT APPLICATION

(11) **EP 4 847 036 A1**
(43) Date of publication of application: **02.12.2026**
(21) Application number: 24923184.6
(22) Date of filing: 17.06.2024
(51) Int. Cl.: A61K 39/00, A61P 37/06, C12N 15/12, C12N 15/864

(54) **METHOD FOR REDUCING OR ELIMINATING ALLOGRAFT REJECTION BY USING THYMUS VACCINE**

(30) Priority: 06.02.2024 CN 202410171327
(71) Applicant: Tongji University, Shanghai 200092 (CN)
(72) Inventor: ZHANG, Xiaoqing, Shanghai 200092 (CN); LIU, Ling, Shanghai 200092 (CN); FENG, Hexi, Shanghai 200092 (CN); LIU, Yang, Shanghai 200092 (CN); LI, Ke, Shanghai 200092 (CN); LI, Ruiyi, Shanghai 200092 (CN)
(74) Representative: Hirons, Daniel Stuart
(86) International application number: PCT/CN2024/099608
(87) International publication number: WO 2025/166964

(57) **Abstract**

Provided is a method for reducing or eliminating allograft rejection by using a thymus vaccine. The method comprises the following steps: expressing major histocompatibility complex (MHC) from a donor and/or grafting a thymus epithelial cell from a donor in the thymus tissue of a recipient by means of the thymus vaccine. The thymus vaccine includes a thymic gene vaccine and/or a thymocyte vaccine. The thymic gene vaccine is used for expressing an MHC antigen from the donor in the thymus of the recipient, and comprises: (1) a gene expression vector; and (2) polynucleotides separately encoding MHC class I molecules and MHC class II molecules of the donor. The thymocyte vaccine is prepared by the following steps: sorting by using an anti-Epcam antibody to give thymus epithelial cells. By establishing a convenient donor source, the limitations of allograft caused by MHC mismatch are overcome, thus helping solve the clinical problem of limited donor sources.

## Description

The present application claims priority to Chinese patent application No. 2024101713279, filed on February 6, 2024. The contents of the above Chinese patent application are incorporated herein by reference in their entireties.

### TECHNICAL FIELD

The present disclosure belongs to the field of biotechnology, and specifically relates to a method for reducing or eliminating allograft rejection using a thymus vaccine, the thymus vaccine (including a thymic gene vaccine or a thymocyte vaccine) used in the method, and the use thereof.

### BACKGROUND

Cell and organ transplantation is considered the ultimate therapeutic approach for the treatment of many organic diseases. Examples include hematopoietic stem cell transplantation for the treatment of hematological disorders, and allogeneic or xenogeneic organ transplantation for the treatment of diseases such as organ failure and necrosis. More than 140,000 patients worldwide undergo organ transplantation each year, primarily involving the kidney, liver, heart, lung, pancreas, and small intestine. In China, the annual demand for organ transplantation exceeds 300,000 cases. However, fewer than 10% of patients who meet the eligibility criteria actually undergo organ transplantation, which is common worldwide. The situation is primarily caused by organ shortage and immune rejection. Although increasing organ donation may help alleviate the shortage of donor organs, immune rejection remains an unresolved challenge ^{[1-2]}.

During allogeneic transplantation, due to the high polymorphism of major histocompatibility complex (MHC) molecules, MHC mismatch between the donor and recipient causes the immune system of the recipient to recognize the graft as "foreign" and initiate an immune attack, leading to rejection ^{[3-4]}. The primary function of MHC molecules is to present antigens to T cells, enabling T cells to recognize and eliminate "foreign" cells. The mechanism by which T cells distinguish "self" from "foreign" is established in the thymus through positive and negative selection ^{[5-6]}. MHC molecules play a critical role in the selection process. In the thymus, the interaction between MHC molecules and T cells determines the fate of developing T cells ^{[7]}. During positive selection, if the affinity between T-cell receptors (TCRs) and MHC molecules is too low, T cells will undergo apoptosis by neglect. During negative selection, if the affinity is too high, T cells will also undergo apoptosis to prevent autoimmunity. Mature T cells that have undergone both positive and negative selection subsequently develop tolerance to "self" ^{[8]}.

Pluripotent stem cell-based therapies and organ transplantation and two major replacement approaches are well-acknowledged ultimate therapeutics for many organic diseases. However, due to the high polymorphism of MHC molecules, when allogeneic transplantation is performed, MHC mismatch between the donor and recipient causes the immune system of the recipient to recognize the graft as "foreign" and initiate an immune attack, leading to rejection. Transplant rejection caused by MHC mismatch remains a major challenge. Although tolerance strategies such as mixed hematopoietic chimerism have been proposed, they are still associated with unstable immune tolerance and a significant risk of graft-versus-host disease (GvHD) ^{[9-12]}. Therefore, there is an urgent need to develop broadly applicable immune tolerance strategies capable of overcoming the limitations of MHC matching. Although MHC mismatch is the principal cause of allograft rejection, the presence of minor histocompatibility antigens may also lead to immune rejection. Accordingly, it is of great significance to develop gene or cell engineering strategies capable of completely overcoming the limitations of MHC matching while enabling the adoptive transfer of minor histocompatibility antigens during allotransplantation.

Gene therapy (GT) employs specific vectors to precisely repair or regulate target genes, thereby altering cellular gene expression patterns to achieve therapeutic effects. Adeno-associated viral (AAV) vectors have been successfully applied in gene therapy. In particular, gene therapy targeting the thymus provides an opportunity to directly intervene in T cell development. For example, Pouzolles et al. have found that intra-thymic (IT) injection of AAV-ZAP-70 into the thymus of ZAP-70-deficient mice led to rapid development of functional T lymphocytes and rapid reconstruction of the thymic medulla. These findings clearly demonstrate the substantial potential and advantages of gene therapy in the treatment of refractory diseases. Technological advances in gene therapy have therefore provided new approaches for addressing the challenge of transplant rejection.

By applying gene expression technologies, the polymorphism of donor-specific antigens makes it difficult to express all antigens from the donor in the thymus of the recipient. Therefore, developing a technology capable of expressing all donor-specific antigens in thymocytes of the recipient, thereby achieving complete adoptive transfer of donor-specific antigens into the thymus of the recipient, is critical for resolving immune rejection during organ transplantation and cell therapy. In thymic tissue, the principal cell types involved in T cell development include thymic epithelial cells and dendritic cells. Expression of donor-specific antigens by dendritic cells and thymic epithelial cells in the recipient thymus for aimed T cell development is an optimized solution.

The current effective strategy for addressing MHC matching is to perform organ transplantation on the basis of the closest possible donor-recipient matching, combined with the administration of immunosuppressants. In addition, scientists have attempted to reduce donor-to-recipient immune rejection by generating donor cells with low immunogenicity (Publication Nos. TW202321435A and CN115335087A). Given the issue of transplant rejection caused by MHC mismatch, developing broadly applicable immune tolerance strategies capable of overcoming the limitations of MHC matching is critical for resolving transplant rejection. Publication No. CN112074277A discloses a technology based on transplantation of cultured allogeneic thymic tissue to induce donor-specific tolerance for allogeneic organ grafts.

### SUMMARY

To address the problem in the prior art that allogeneic transplantation imposes significant limitations on the donor, the present disclosure provides a method for reducing or eliminating allograft rejection using a thymus vaccine, the thymus vaccine (including a thymic gene vaccine and a thymocyte vaccine) used in the method, and the use thereof.

In the prior art, donor-derived thymic tissue is cultured and transplanted into muscle tissue to establish a new simulated thymus, thereby achieving donor-specific tolerance to donor-derived grafts. However, the technology imposes significant limitations on the donor. T cells undergo a complex process of development and maturation in the thymus, primarily including negative selection and positive selection, through which self-reactive T cells are eliminated. Mature T cells that have undergone the selection process, which is primarily determined by self-antigens, establish immune tolerance to self. Thus, when T cells are stimulated by foreign antigens during the selection process, whether T cells reactive to such foreign antigens are eliminated after positive and negative selection, *i.e.*, whether T cells that do not attack cells expressing the corresponding foreign antigens can be generated, is a critical issue in allogeneic transplantation.

The present disclosure simulates the natural process by which T cells establish self-tolerance, using AAV vectors with low immunogenicity to express donor-derived allogeneic MHC (allo-MHC) molecules in the thymus of the recipient. Following positive and negative selection, donor-reactive T cells undergo apoptosis, whereas mature T cells recognize cells expressing either self-MHC or allo-MHC as "self", thereby establishing long-term and stable donor-specific tolerance.

In addition, the success of allogeneic cell or organ transplantation depends primarily on histocompatibility, which is determined by histocompatibility antigens. Histocompatibility antigens are classified into major histocompatibility antigens and minor histocompatibility antigens. Antigens responsible for acute rejection are referred to as mismatched major histocompatibility antigens. However, immune rejection has also been observed in organ transplantation between monozygotic twins sharing identical major histocompatibility antigens, primarily due to mismatched minor histocompatibility antigens. The thymic gene vaccine strategy, which expresses donor-derived major histocompatibility antigens in thymic epithelial cells, can resolve immune rejection during cell or organ transplantation. However, the strategy remains insufficient in cases where immune rejection is caused by minor histocompatibility antigens.

Accordingly, the present disclosure, for the first time, proposes co-expression of donor-derived major histocompatibility antigens and minor histocompatibility antigens in the thymus of the recipient. The polymorphism of minor histocompatibility antigens limits their current expression using transgenic technologies. However, transplantation of donor-derived thymic epithelial cells into the thymus of the recipient enables complete expression of donor histocompatibility antigens in the thymus of the recipient.

To overcome the limitations of MHC matching and to enable adoptive transfer of polymorphic minor histocompatibility antigens, the present disclosure has developed a thymus vaccine strategy for adoptive transfer of donor-derived antigens into the thymus of the recipient, which includes: 1) expression of donor-derived MHC molecules in the thymus of the recipient via adenoviral infection; 2) transplantation of donor-derived thymic epithelial cells into the thymus of the recipient to achieve expression of donor-derived MHC molecules and minor histocompatibility antigens in the thymus of the recipient.

Accordingly, a first technical solution of the present disclosure provides a method for reducing or eliminating allograft rejection, wherein the method comprises the following steps:
expressing donor-derived MHC and/or transplanting donor-derived thymic epithelial cells in the thymic tissue of a recipient using a thymus vaccine; the thymus vaccine comprises a thymic gene vaccine and/or a thymocyte vaccine.

Preferably, the method is performed for non-therapeutic purposes; and/or, the method is performed *in vitro* or *ex vivo*.

In some preferred embodiments, the expression of MHC from the donor is achieved by injecting the thymic gene vaccine into the thymic tissue of the recipient.

Preferably, the thymic gene vaccine comprises:
(1) a gene expression vector;
(2) polynucleotides encoding donor-derived MHC class I molecules and MHC class II molecules, respectively;
wherein the polynucleotides are operably linked to the gene expression vector, and the MHC background of the donor is different from that of the recipient.

More preferably, the gene expression vector comprises a plasmid vector and a viral vector; and/or, the polynucleotides encoding donor-derived MHC class I molecules and MHC class II molecules are contained within a single gene expression vector or within two separate gene expression vectors.

In some preferred embodiments, the viral vector is a lentiviral vector or an adeno-associated viral (AAV) vector, such as AAV2/8.

Preferably, the thymic gene vaccine comprises AAV-CMV-H2-Kb α chain-IRES-H2-Db α chain and AAV-CMV-I-Ab α chain-IRES-β chain.

More preferably, the thymic gene vaccine comprises isolated nucleic acids having the nucleotide sequences as set forth in SEQ ID NO: 1 and SEQ ID NO: 2, respectively; and/or, the thymic gene vaccine is injected into the left and right lobes of the thymus of the recipient, at 1 to 5 sites in each lobe, such as 3 sites.

In some more preferred embodiments, following injection of the thymic gene vaccine, newly matured T cells in the thymus of the recipient are educated by both self-MHC and the MHC expressed by the thymus vaccine, thereby eliminating donor-reactive T cells.

Preferably, prior to injection of the thymus vaccine, the method further comprises the following steps:
(i) injecting or not injecting IFN-γ;
(ii) injecting a T cell depleting agent or an immunosuppressant; for example, the T cell depleting agent is antibodies 2.43 and GK1.5;
(iii) irradiation; for example, irradiation at 1.5 to 3 Gy for 1 to 2 minutes on the second day.

As used herein, administration of IFN-γ in mice rapidly induces high levels of MHC expression. When the recipient is a human, injection of IFN-γ is unnecessary since IFN-γ can be produced by the body.

As used herein, T cell depletion is performed in mouse experiments. In human organ transplantation, administration of immunosuppressants as part of basic therapy can effectively prevent graft rejection. An ideal immunosuppressive therapy should both prevent graft rejection and avoid toxic side effects to the recipient. Commonly used immunosuppressants include azathioprine, mycophenolate mofetil, corticosteroids, cyclosporine, tacrolimus, antilymphocyte globulin, muromonab-CD3, *etc*.

Accordingly, a second technical solution of the present disclosure provides a method for tissue or organ transplantation, comprising the following steps:
A. reducing or eliminating allograft rejection using the method according to the first technical solution;
B. performing cell, tissue, or organ transplantation before or after peripheral blood T cells of the recipient has returned to normal levels;
   preferably, the tissue or organ is selected from skin, islet, nerve, kidney, liver, heart, small intestine, fat, and bone, any one or any combination thereof.

The thymus vaccine strategy of the present disclosure comprises two principal aspects. The first aspect is based on gene editing technology to express donor-derived MHC molecules in the thymus of the recipient, thereby overcoming the limitations of MHC matching. The gene therapy-based strategy opens a new path for the application of gene therapy in the field of transplantation immunology. Gene therapy has already demonstrated great potential in the treatment of numerous diseases. By employing specific vectors, such as adeno-associated virus (AAV), gene therapy enables the introduction of specific genes into target cells, thereby altering their gene expression patterns to achieve therapeutic effects. The second aspect involves transplantation of allogeneic thymic epithelial cells into the thymus of the recipient. Since these donor-derived thymic epithelial cells carry all the antigenic information of the donor, the strategy not only overcomes MHC mismatch but also prevents immune rejection that may be caused by minor histocompatibility antigens.

Accordingly, a third technical solution of the present disclosure provides a thymic gene vaccine for expressing donor-derived major histocompatibility complex (MHC) antigens in the thymus of a recipient, comprising:
(1) a gene expression vector;
(2) polynucleotides encoding donor-derived MHC class I molecules and MHC class II molecules, respectively;
wherein the polynucleotides are operably linked to the gene expression vector, and the MHC background of the donor is different from that of the recipient.

Preferably, the gene expression vector comprises a plasmid vector and a viral vector; and/or, the polynucleotides encoding donor-derived MHC class I molecules and MHC class II molecules are contained within a single gene expression vector or within separate gene expression vectors.

In some preferred embodiments, the viral vector is a lentiviral vector or an adeno-associated viral (AAV) vector, such as AAV2/8.

Preferably, the thymic gene vaccine comprises AAV-CMV-H2-Kb α chain-IRES-H2-Db α chain and AAV-CMV-I-Ab α chain-IRES-β chain.

More preferably, the thymic gene vaccine comprises isolated nucleic acids having the nucleotide sequences as set forth in SEQ ID NO: 1 and SEQ ID NO: 2, respectively.

A fourth technical solution of the present disclosure provides a thymocyte vaccine prepared by the following steps: isolating thymic epithelial cells using an anti-Epcam antibody to obtain the thymocyte vaccine.

Preferably, the thymic epithelial cells are administered at a dose of 2 × 10⁶ cells per recipient; and/or, the thymic epithelial cells are suspended in phosphate-buffered saline (PBS) to prepare the thymocyte vaccine.

When the recipient is a human, the amount of thymic epithelial cells transplanted is approximately 50% of the total number of human thymocytes based on the corresponding mouse-to-human ratio.

In the present disclosure, donor-derived thymic epithelial cells are isolated and transplanted into the thymus of the recipient. According to the present disclosure, those skilled in the art can achieve the effect of resolving immune rejection by transplanting donor-derived thymic epithelial cells obtained in any form (*e.g.*, primary cells or cells differentiated from pluripotent stem cells) into the thymus of the recipient.

A fifth technical solution of the present disclosure provides a thymocyte vaccine kit comprising the thymic gene vaccine according to the third technical solution or the thymocyte vaccine according to the fourth technical solution;
optionally further comprising: (i) IFN-γ; and/or
(ii) a T cell depleting agent or an immunosuppressant.

A sixth technical solution of the present disclosure provides the use of the thymic gene vaccine according to the third technical solution, the thymocyte vaccine according to the fourth technical solution, or the thymocyte vaccine kit according to the fifth technical solution in the manufacture of an immune preparation for reducing or eliminating allograft rejection, or in reducing or eliminating allograft rejection.

On the basis of common knowledge in the art, the preferred conditions described above may be arbitrarily combined to obtain preferred examples of the present disclosure.

The reagents and raw materials used in the present disclosure are all commercially available.

The positive and progressive effects of the present disclosure are as follows:

The present disclosure establishes a transplantation tolerance strategy based on thymus vaccines, providing a convenient and universally applicable donor source and overcoming the limitations of allograft transplantation caused by MHC mismatch, thereby facilitating the resolution of donor availability issues in clinical practice. The gene therapy-based strategy opens a new path for the application of gene therapy in the field of transplantation immunology.

### BRIEF DESCRIPTION OF THE DRAWINGS

FIG. 1 is a schematic diagram of the plasmid constructs; Panel A shows the construction of the thymic gene vaccine plasmid encoding the donor MHC β chain, in which the H2^{Kb} and H2^{Db} gene fragments of the donor MHC β chain are inserted into the PGMAAV-4878 expression vector; Panel B shows the construction of the thymic gene vaccine plasmid encoding the donor MHC α chain, in which the Iα (I-A) gene fragment of the donor MHC α chain is inserted into the PGMAAV-4878 expression vector.
FIG. 2 shows the expression of the thymic gene vaccine in thymic epithelial cells of the recipient.
FIG. 3 shows the expression of the thymic gene vaccine in dendritic cells within the thymus of the recipient.
FIG. 4 shows the elimination of donor-reactive T cells following administration of the thymic gene vaccine.
FIG. 5 shows the results of donor skin transplantation following administration of the thymic gene vaccine.
FIG. 6 shows that donor islet transplantation following administration of the thymic gene vaccine can effectively treat type I diabetes.
FIG. 7 shows teratoma formation in allogeneic mice following administration of the thymic gene vaccine.
FIG. 8 shows the growth of multiple organs and tissues following administration of the thymic gene vaccine.
FIG. 9 shows the isolation results of the thymocyte vaccine.
FIG. 10 shows the long-term survival of the thymocyte vaccine in the thymus of the recipient.
FIG. 11 shows the results of donor skin transplantation following administration of the thymocyte vaccine.
FIG. 12 shows that donor islet transplantation following administration of the thymocyte vaccine can effectively treat type I diabetes.

### DETAILED DESCRIPTION OF THE PREFERRED EMBODIMENT

The present disclosure is further illustrated below by way of examples, but is not thereby limited to the scope of the described examples. Experimental methods for which specific conditions are not indicated in the following examples are selected according to conventional methods and conditions, or according to the product instructions.

### Example 1: Thymic gene vaccine

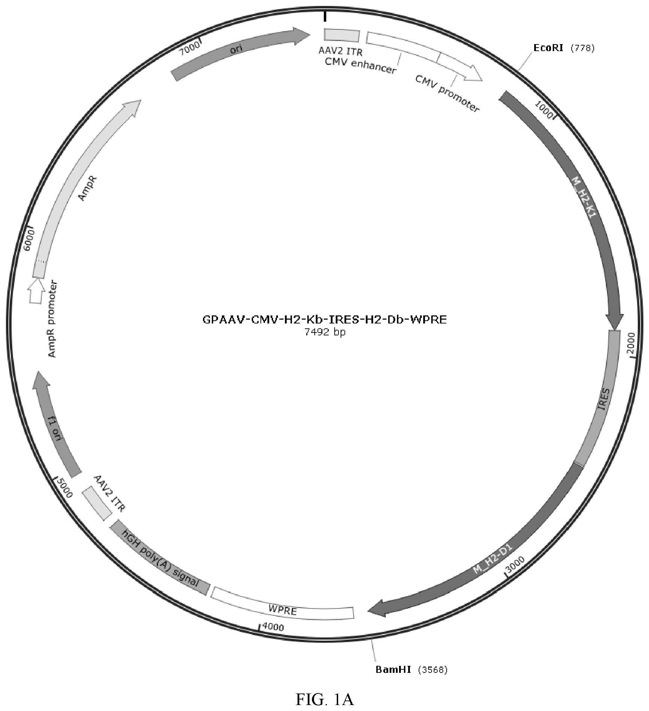

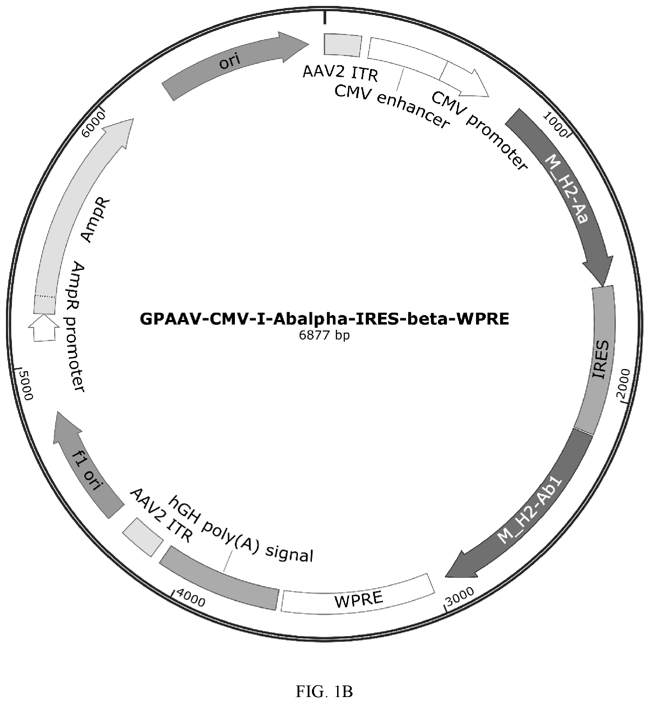

To achieve expression of allo-MHC molecules in the thymus of the recipient, recombinant AAV vectors were constructed based on the gene sequences encoding the donor-derived MHC molecules. First, a pair of mice with completely mismatched MHC haplotypes was selected, in which Balb/c mice carrying the H2d MHC haplotype served as recipients and C57 mice carrying the H2b MHC haplotype served as donors. Subsequently, based on the MHC gene sequences of the donor mice, recombinant AAV vectors expressing H2b class I and class II molecules were constructed, namely AAV2/8-CMV-H2-Kb α chain-IRES-H2-Db α chain and AAV2/8-CMV-I-Ab α chain-IRES-β chain (collectively referred to as the thymic gene vaccine), as shown in Panels A and B of FIG. 1, respectively. The corresponding nucleotide sequences were designated GPAAV-CMV-H2-Kb-IRES-H2-Db-WPRE and GPAAV-CMV-I-Ab-alpha-IRES-beta-WPRE, respectively.
Nucleotide sequence of **GPAAV-CMV-H2-Kb-IRES-H2-Db-WPRE** 
GAATTCGCCACCATGGTACCGTGCACGCTGCTCCTGCTGTTGGCGGCCGC
CCTGGCTCCGACTCAGACCCGCGCGGCCCACACTCGCTGAGGTATTTCGTCACC
GCCGTGTCCCGGCCCGGCCTCGGGGAGCCCCGGTACATGGAAGTCGGCTACGTGG
ACGACACGGAGTTCGTGCGCTTCGACAGCGACGCGGAGAATCCGAGATATGAGCC
 
GCGGGCGCGGTGGATGGAGCAGGAGGGGCCCGAGTATTGGGAGCGGAGACACA
GAAAGCCAAGGGCAATGAGCAGAGTTTCCGAGTGGACCTGAGGACCCTGCTCGG
CTACTACAACCAGAGCAAGGGCGGCTCTCACACTATTCAGGTGATCTCTGGCTGT
GAAGTGGGGTCCGACGGGCGACTCCTCCGCGGGTACCAGCAGTACGCCTACGAC
GGCTGCGATTACATCGCCCTGAACGAAGACCTGAAAACGTGGACGGCGGCGGAC
ATGGCGGCGCTGATCACCAAACACAAGTGGGAGCAGGCTGGTGAAGCAGAGAGA
CTCAGGGCCTACCTGGAGGGCACGTGCGTGGAGTGGCTCCGCAGATACCTGAAG
AACGGGAACGCGACGCTGCTGCGCACAGATTCCCCAAAGGCCCATGTGACCCATC
ACAGCAGACCTGAAGATAAAGTCACCCTGAGGTGCTGGGCCCTGGGCTTCTACCC
TGCTGACATCACCCTGACCTGGCAGTTGAATGGGGAGGAGCTGATCCAGGACATG
GAGCTTGTGGAGACCAGGCCTGCAGGGGATGGAACCTTCCAGAAGTGGGCATCT
GTGGTGGTGCCTCTTGGGAAGGAGCAGTATTACACATGCCATGTGTACCATCAGGG
GCTGCCTGAGCCCCTCACCCTGAGATGGGAGCCTCCTCCATCCACTGTCTCCAAC
ATGGCGACCGTTGCTGTTCTGGTTGTCCTTGGAGCTGCAATAGTCACTGGAGCTGT
GGTGGCTTTTGTGATGAAGATGAGAAGGAGAAACACAGGTGGAAAAGGAGGGGA
CTATGCTCTGGCTCCAGGCTCCCAGACCTCTGATCTGTCTCTCCCAGATTGTAAAG
TGATGGTTCATGACCCTCATTCTCTAGCGTGACCCCTCTCCCTCCCCCCCCCTAAC
GTTACTGGCCGAAGCCGCTTGGAATAAGGCCGGTGTGCGTTTGTCTATATGTTATTT
TCCACCATATTGCCGTCTTTTGGCAATGTGAGGGCCCGGAAACCTGGCCCTGTCTT
CTTGACGAGCATTCCTAGGGGTCTTTCCCCTCTCGCCAAAGGAATGCAAGGTCTGT
TGAATGTCGTGAAGGAAGCAGTTCCTCTGGAAGCTTCTTGAAGACAAACAACGTC
TGTAGCGACCCTTTGCAGGCAGCGGAACCCCCCACCTGGCGACAGGTGCCTCTGC
GGCCAAAAGCCACGTGTATAAGATACACCTGCAAAGGCGGCACAACCCCAGTGCC
ACGTTGTGAGTTGGATAGTTGTGGAAAGAGTCAAATGGCTCACCTCAAGCGTATT
CAACAAGGGGCTGAAGGATGCCCAGAAGGTACCCCATTGTATGGGATCTGATCTG
GGGCCTCGGTGCACATGCTTTACATGTGTTTAGTCGAGGTTAAAAAACGTCTAGGC
CCCCCGAACCACGGGGACGTGGTTTTCCTTTGAAAAACACGATGATAAGCCACCA
TGGGGGCGATGGCTCCGCGCACGCTGCTCCTGCTGCTGGCGGCCGCCCTGGCCCC
GACTCAGACCCGCGCGGGCCCACACTCGATGCGGTATTTCGAGACCGCCGTGTCC
CGGCCCGGCCTCGAGGAGCCCCGGTACATCTCTGTCGGCTATGTGGACAACAAGG
 
AGTTCGTGCGCTTCGACAGCGACGCGGAGAATCCGAGATATGAGCCGCGGGCGCC
GTGGATGGAGCAGGAGGGGCCGGAGTATTGGGAGCGGAAACACAGAAAGCCA
AGGGCCAAGAGCAGTGGTTCCGAGTGAGCCTGAGGAACCTGCTCGGCTACTACA
ACCAGAGCGCGGGCGGCTCTCACACACTCCAGCAGATGTCTGGCTGTGACTTGGG
GTCGGACTGGCGCCTCCTCCGCGGGTACCTGCAGTTCGCCTATGAAGGCCGCGAT
TACATCGCCCTGAACGAAGACCTGAAAACGTGGACGGCGGCGGACATGGCGGCG
CAGATCACCCGACGCAAGTGGGAGCAGAGTGGTGCTGCAGAGCATTACAAGGCC
TACCTGGAGGGCGAGTGCGTGGAGTGGCTCCACAGATACCTGAAGAACGGGAAC
GCGACGCTGCTGCGCACAGATTCCCCAAAGGCACATGTGACCCATCACCCCAGAT
CTAAAGGTGAAGTCACCCTGAGGTGCTGGGCCCTGGGCTTCTACCCTGCTGACAT
CACCCTGACCTGGCAGTTGAATGGGGAGGAGCTGACCCAGGACATGGAGCTTGT
GGAGACCAGGCCTGCAGGGGATGGAACCTTCCAGAAGTGGGCATCTGTGGTGGT
GCCTCTTGGGAAGGAGCAGAATTACACATGCCGTGTGTACCATGAGGGGCTGCCT
GAGCCCCTCACCCTGAGATGGGAGCCTCCTCCGTCCACTGACTCTTACATGGTGAT
CGTTGCTGTTCTGGGTGTCCTTGGAGCTATGGCCATCATTGGAGCTGTGGTGGCTT
TTGTGATGAAGAGAAGGAGAAACACAGGTGGAAAAGGAGGGGACTATGCTCTGG
CTCCAGGCTCCCAGAGCTCTGAAATGTCTCTCCGAGATTGTAAAGCGTGAGGATC
C (SEQ ID NO: 1)
Nucleotide sequence of **GPAAV-CMV-I-Ab-alpha-IRES-beta-WPRE**

GCCACCATGCCGCGCAGCAGAGCTCTGATTCTGGGGGTCCTCGCCCTGAC
CACCATGCTCAGCCTCTGTGGAGGTGAAGACGACATTGAGGCCGACCACGTAGGC
ACCTATGGTATAAGTGTATATCAGTCTCCTGGAGACATTGGCCAGTACACATTTGAA
TTTGATGGTGATGAGTTGTTCTATGTGGACTTGGATAAGAAGGAGACTGTCTGGAT
GCTTCCTGAGTTTGGCCAATTGGCAAGCTTTGACCCCCAAGGTGGACTGCAAAAC
ATAGCTGTAGTAAAACACAACTTGGGAGTCTTGACTAAGAGGTCAAATTCCACCC
CAGCTACCAATGAGGCTCCTCAAGCGACTGTGTTCCCCAAGTCCCCTGTGCTGCT
GGGTCAGCCCAACACCCTCATCTGCTTTGTGGACAACATCTTCCCTCCTGTGATCA
ACATCACATGGCTCAGAAATAGCAAGTCAGTCGCAGACGGTGTTTATGAGACCAG
CTTCTTCGTCAACCGTGACTATTCCTTCCACAAGCTGTCTTATCTCACCTTCATCCC
TTCTGACGATGACATTTATGACTGCAAGGTGGAACACTGGGGCCTGGAGGAGCCG

GTTCTGAAACACTGGAACCTGAGATTCCAGCCCCCATGTCAGAGCTGACAGAGA
CTGTGGTCTGTGCCCTGGGGTTGTCTGTGGCCTTGTGGCATCGTGGTGGGCAC
CATCTTCATCATTCAAGGCCTGCGATCAGGTGGCACCTCCAGACACCCAGGGCCTT
TATGACCCCTCTCCCTCCCCCCCCCTAACGTTACTGGCCGAAGCCGCTTGGAATA
AGGCCGGTGTGCGTTTGTCTATATGTTATTTTCCACCATATTGCCGTCTTTTGGCAAT
GTGAGGGCCCGGAAACCTGGCCCTGTCTTCTTGACGAGCATTCCTAGGGGTCTTT
CCCCTCTCGCCAAAGGAATGCAAGGTCTGTTGAATGTCGTGAAGGAAGCAGTTCC
TCTGGAAGCTTCTTGAAGACAAACAACGTCTGTAGCGACCCTTTGCAGGCAGCGG
AACCCCCCACCTGGCGACAGGTGCCTCTGCGGCCAAAAGCCACGTGTATAAGATA
CACCTGCAAAGGCGGCACAACCCCAGTGCCACGTTGTGAGTTGGATAGTTGTGGA
AAGAGTCAAATGGCTCACCTCAAGCGTATTCAACAAGGGGCTGAAGGATGCCCAG
AAGGTACCCCATTGTATGGGATCTGATCTGGGGCCTCGGTGCACATGCTTTACATG
TGTTTAGTCGAGGTTAAAAAACGTCTAGGCCCCCCGAACCACGGGGACGTGGTTT
TCCTTTGAAAAACACGATGATAAGCCACCATGGCTCTGCAGATCCCCAGCCTCCTC
CTCTCGGCTGCTGTGGTGGTGCTGATGGTGCTGAGCAGCCCAGGGACTGAGGGCG
GAGACTCCGAAAGGCATTTCGTGTACCAGTTCATGGGCGAGTGCTACTTCACCAA
CGGGACGCAGCGCATACGATATGTGACCAGATACATCTACAACCGGGAGGAGTAC
GTGCGCTACGACAGCGACGTGGGCGAGCACCGCGCGGTGACCGAGCTGGGGCGG
CCAGACGCCGAGTACTGGAACAGCCAGCCGGAGATCCTGGAGCGAACGCGGGCC
GAGCTGGACACGGTGTGCAGACACAACTACGAGGGGCCGGAGACCCACACCTCC
CTGCGGCGGCTTGAACAGCCCAATGTCGTCATCTCCCTGTCCAGGACAGAGGCCC
TCAACCACCACAACACTCTGGTCTGCTCAGTGACAGATTTCTACCCAGCCAAGAT
CAAAGTGCGCTGGTTCCGGAATGGCCAGGAGGAGACGGTGGGGGTCTCATCCAC
ACAGCTTATTAGGAATGGGGACTGGACCTTCCAGGTCCTGGTCATGCTGGAGATG
ACCCCTCGGCGGGGAGAGGTCTACACCTGTCACGTGGAGCATCCCAGCCTGAAG
AGCCCCATCACTGTGGAGTGGAGGGCACAGTCTGAGTCTGCCTGGAGCAAGATGT
TGAGCGGCATCGGGGGCTGCGTGCTTGGGGTGATCTTCCTCGGGCTTGGCCTTTTC
ATCCGTCACAGGAGTCAGAAAGGACCTCGAGGCCCTCCTCCAGCAGGGCTCCTG
CAGTGA (SEQ ID NO: 2)

First, 400 µL of a T cell depleting antibody mixture, including antibodies 2.43 (BioXCell, Cat. No. BE0061) and GK1.5 (BioXCell, Cat. No. BP0003-1), was administered intraperitoneally to 8-week-old Balb/C mice. Specifically, antibody GK1.5 was used at a concentration of 9.79 mg/mL, with 40.8 µL per tube; antibody 2.43 was used at a concentration of 9.9 mg/mL, with 40.4 µL per tube. On Day 2, T cell depletion was performed by subjecting the mice to irradiation at 1.5 Gy for 1 minute. One week later, the thymic gene vaccine was injected into the thymus of Balb/C mice that had undergone T cell depletion, with three injection sites (upper, middle, and lower regions) in each of the left and right lobes. Following successful vaccination, newly matured T cells in the thymus of the recipient mice were educated by both self-MHC molecules and the MHC molecules expressed by the thymic gene vaccine, thereby eliminating donor-reactive T cells. Forty-two days later, after peripheral blood T cells of the recipient mice had returned to normal levels, transplantation experiments using C57-derived skin, islet, kidney, liver, and heart were performed.

Four weeks after skin transplantation, peripheral blood was collected from the recipient mice. CD3-positive T cells were isolated by flow cytometry for scRNA-seq and TCR-seq analysis. Single-cell sequencing demonstrated that the technology described herein achieved clonal elimination of donor-reactive T cells, and the educated T cells no longer attacked the recipient.

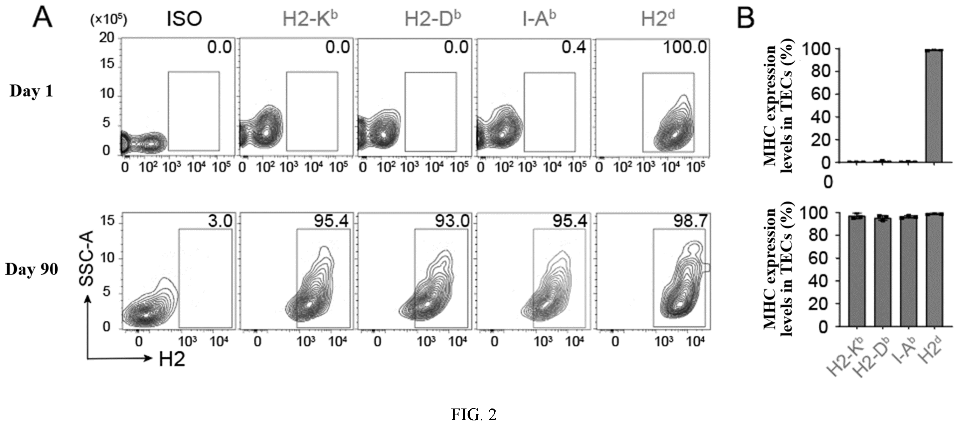

Following intrathymic administration of the donor-derived thymic gene vaccine, the transduction efficiency of the thymic gene vaccine in thymic epithelial cells of the recipient was evaluated by flow cytometry. After infection with the thymic gene vaccine, thymic epithelial cells continuously expressed donor-derived MHC molecules for an extended period and maintained high levels of expression on Day 90 (see Panels A and B of FIG. 2).

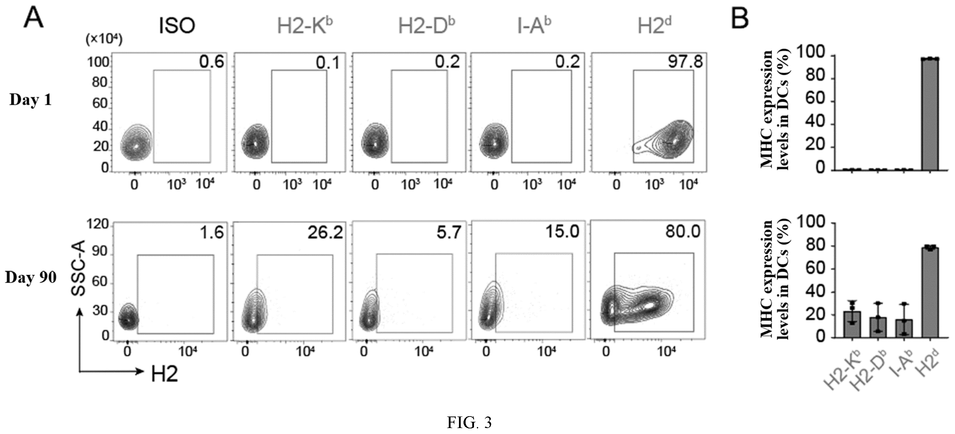

Following intrathymic administration of the donor-derived thymic gene vaccine, the transduction efficiency of the thymic gene vaccine in dendritic cells within the thymus of the recipient was evaluated by flow cytometry. After infection with the thymic gene vaccine, dendritic cells in the thymus continuously expressed donor-derived MHC molecules for an extended period and maintained high levels of expression on Day 90, although a slight reduction in expression was observed, which may be attributed to the migratory nature of dendritic cells (see Panels A and B of FIG. 3).

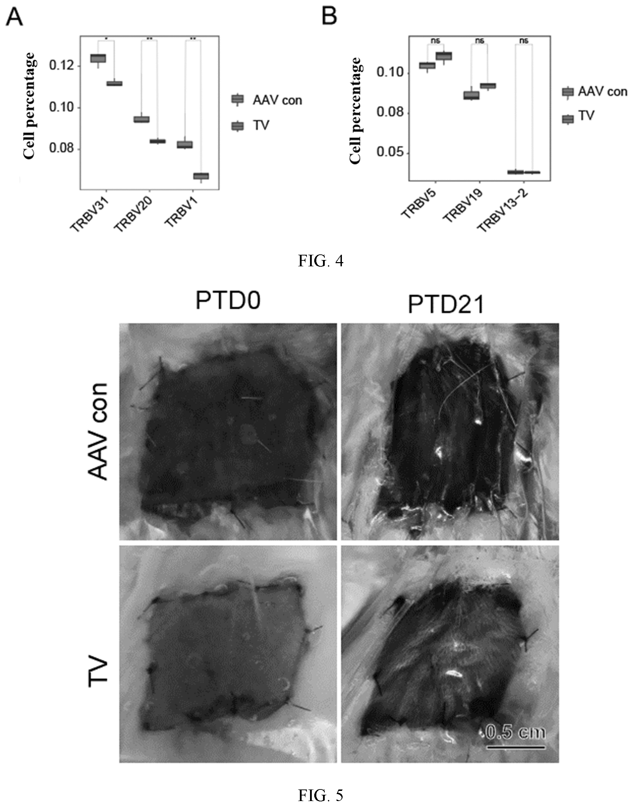

Following administration of the thymic gene vaccine, all newly matured T cells were tolerant to both the recipient and the donor. TCR expression in newly matured T cells was analyzed by single-cell TCR sequencing. Representative TCRs exhibiting relatively high levels of expression, including TRBV5, TRBV19, TRBV13-2, TRBV31, TRBV20, and TRBV1, were analyzed. The results demonstrated that expression levels of the donor-reactive TCRs TRBV31, TRBV20, and TRBV1 decreased following administration with the thymic gene vaccine, indicating that donor-reactive T cells were eliminated (see Panels A and B of FIG. 4).

Due to the elimination of donor-reactive T cells following administration of the thymic gene vaccine, newly matured T cells of the recipient no longer attacked donor-derived cells, tissues, and organs. The efficacy of the vaccine was evaluated using skin transplantation, which is considered the most challenging. The results demonstrated that donor skin grafts survived well after transplantation into the recipient (see FIG. 5); wherein "PTD0" and "PTD0" represent Days 0 and 21 post-transplantation, respectively.

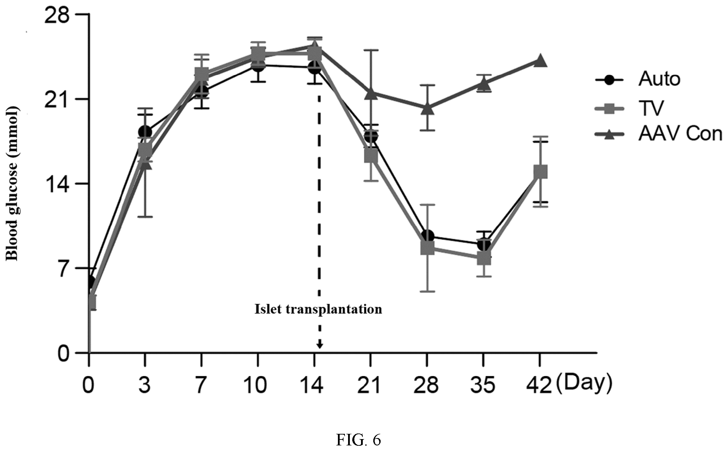

Type 1 diabetes models were established in wild-type C57 mice, wild-type Balb/c mice, and Balb/c mice treated with the thymic gene vaccine. After successful establishment of the diabetic mouse models, C57-derived islets were transplanted beneath the renal capsule of diabetic mice in three groups, and changes in blood glucose levels were monitored at different time points. Following islet autotransplantation (Auto) in C57 mice, blood glucose levels in the model mice significantly decreased and approached normal levels within three weeks, but gradually increased after the fourth week, which was attributed to functional exhaustion of the transplanted islets caused by excessive insulin production. In the thymic AAV empty-vector control group (AAV Con), blood glucose levels slightly decreased within two weeks after islet transplantation, which was attributed to the limited function of the transplanted islets before they were completely rejected. In contrast, blood glucose levels in the thymic gene vaccine group (TV) after islet transplantation were comparable to those observed in the islet autotransplantation group. These results demonstrated that, following administration of the thymic gene vaccine, allogeneic islet transplantation can effectively treat type I diabetes, as shown in FIG. 6.

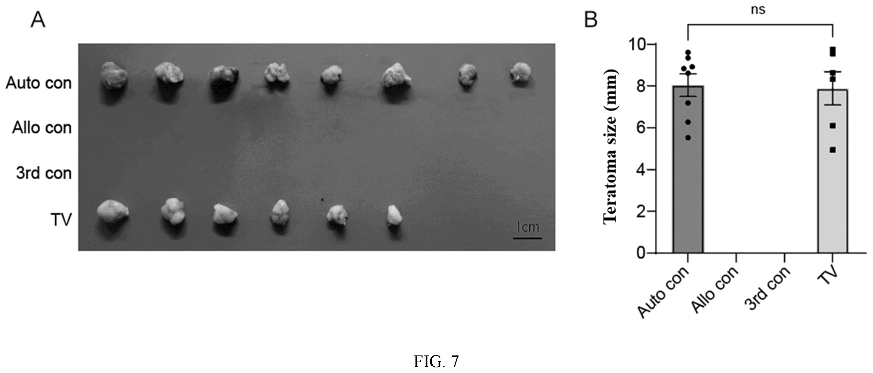

To further verify the allogeneic effect, C57 mouse embryonic stem cells were injected subcutaneously into C57 mice, Balb/c mice, C3H mice, and Balb/c mice treated with the thymic gene vaccine for teratoma formation assays. The results demonstrated that teratomas developed successfully in both the C57 group (Auto con) and the thymic gene vaccine-treated Balb/c group (TV), and no significant difference in teratoma size was observed between the two groups. In contrast, no teratomas developed in the Balb/c group (Allo con) or the C3H group (3rd con), as shown in Panels A and B of FIG. 7.

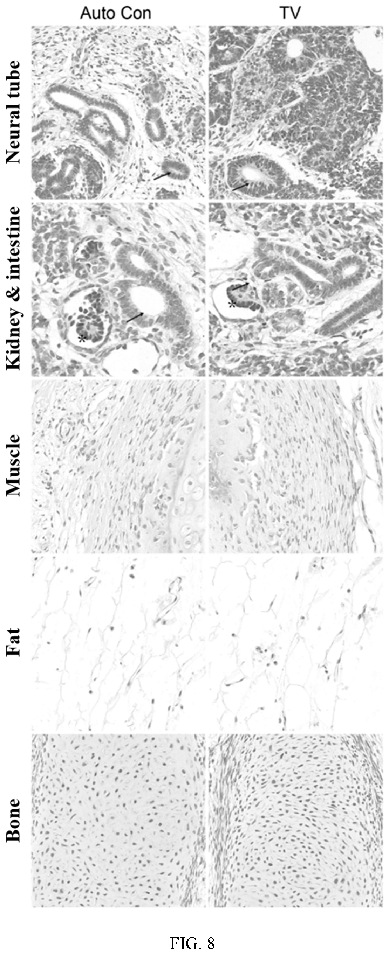

Pluripotent stem cells were transplanted subcutaneously into the C57 group (Auto con) and the thymic gene vaccine-treated Balb/c group (TV). The results demonstrated that multiple organs and tissues developed successfully in both groups, indicating the beneficial effects of the thymic gene vaccine on the allogeneic transplantation and growth of multiple organs. These findings confirmed that the thymic gene vaccine can effectively enable allogeneic transplantation of multiple organs, as shown in FIG. 8.

### Example 2: Thymocyte vaccine

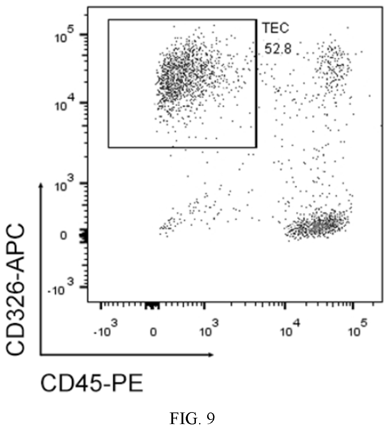

Male and female C57BL/6JSmoc-Gt(ROSA)26S or em1(CAG-Luc-EGFP)Smoc mice were mated to obtain neonatal (P1-P7) mice. Thymic epithelial cells were isolated by magnetic bead sorting using an anti-Epcam mAb, thereby obtaining the thymocyte vaccine, as shown in FIG. 9. Specifically, thymic epithelial cells were isolated from the thymuses of neonatal (P1-P7) C57 mice based on the surface marker CD326. The isolated thymic epithelial cells were suspended in PBS containing Matrigel and 10% FBS. A total of 2 × 10⁶ thymic epithelial cells per recipient were transplanted into the left and right lobes of the thymus of recipient mice that had undergone T cell depletion (as described in Example 1). At each injection site, 1 × 10⁶ cells suspended in 5 µL of PBS were administered. Donor-derived thymic epithelial cells transplanted into the thymus of the recipient were monitored by luciferase bioluminescence imaging and remained detectable 90 days after transplantation.

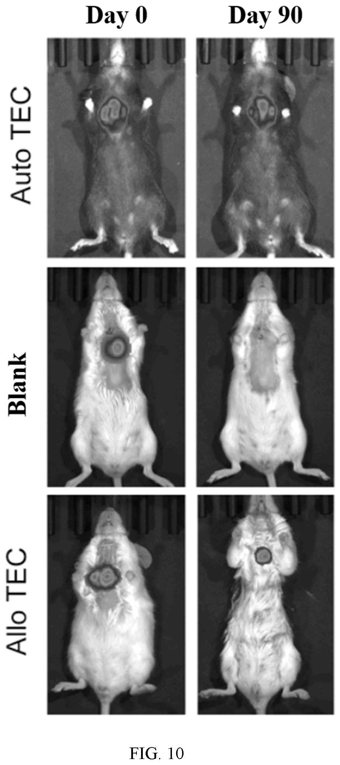

The resulting thymocyte vaccine was transplanted into the thymus of the recipient. An isotype control group and a control group without prior T cell depletion were included. The results demonstrated that the transplanted thymocyte vaccine survived long-term in the thymus of the recipient (see FIG. 10).

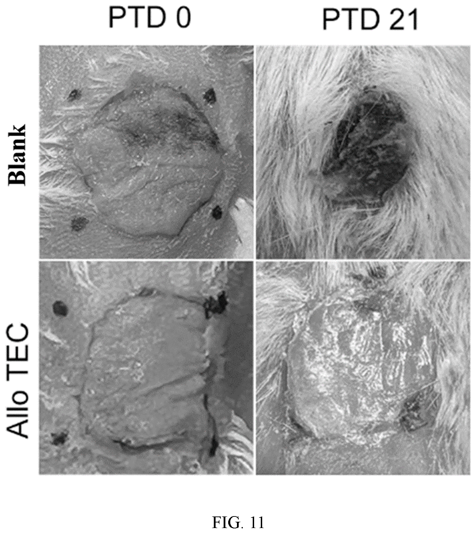

Following administration of the thymocyte vaccine, the efficacy of the thymocyte vaccine was evaluated using skin transplantation, which is considered the most challenging. The results demonstrated that donor skin grafts survived well after transplantation into the recipient (see FIG. 11); wherein "PTD0" and "PTD0" represent Days 0 and 21 post-transplantation, respectively.

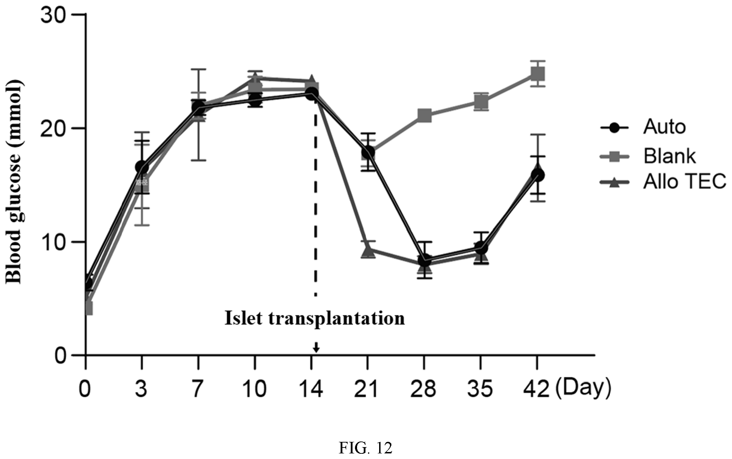

Type 1 diabetes models were established in wild-type C57 mice, sham-operated wild-type Balb/c mice, and Balb/c mice treated with the thymocyte vaccine. After successful establishment of the diabetic mouse models, C57-derived islets were transplanted beneath the renal capsule of diabetic mice in three groups, and changes in blood glucose levels were monitored at different time points. Following islet autotransplantation (Auto) in C57 mice, blood glucose levels in the model mice significantly decreased and approached normal levels within three weeks, but gradually increased after the fourth week, which was attributed to functional exhaustion of the transplanted islets caused by excessive insulin production. In the thymic sham-operated group (Blank), blood glucose levels slightly decreased within two weeks after islet transplantation, which was attributed to the limited function of the transplanted islets before they were completely rejected. In contrast, blood glucose levels in the thymocyte vaccine group (Allo TEC) after islet transplantation were comparable to those observed in the islet autotransplantation group. These results demonstrated that, following administration of the thymocyte vaccine, allogeneic islet transplantation can effectively treat type I diabetes, as shown in FIG. 12.

### References

[1] Global activity in organ transplantation estimations.
[2] Report on Organ Transplantation Development in China (book).
[3] Montgomery, R. A., Tatapudi, V. S., Leffell, M. S. & Zachary, A. A. HLA in transplantation. Nat Rev Nephrol 14, 558-570, doi:10.1038/s41581-018-0039-x (2018).
[4] Bodmer, J. G. et al. Nomenclature for factors of the HLA system, 1998. Tissue Antigens 53, 407-446, doi:10.1034/j.1399-0039.1999.530421.x (1999).
[5] Zachary, A. A. & Leffell, M. S. HLA Mismatching Strategies for Solid Organ Transplantation - A Balancing Act. Front Immunol 7, 575, doi:10.3389/fimmu.2016.00575 (2016).
[6] Williams, R. C., Opelz, G., McGarvey, C. J., Weil, E. J. & Chakkera, H. A. The Risk of Transplant Failure With HLA Mismatch in First Adult Kidney Allografts From Deceased Donors. Transplantation 100, 1094-1102, doi:10.1097/TP.0000000000001115 (2016).
[7] Jameson, S. C., Hogquist, K. A. & Bevan, M. J. Positive selection of thymocytes. Annu Rev Immunol 13, 93-126, doi:10.1146/annurev.iy.13.040195.000521 (1995).
[8] Goldrath, A. W. & Bevan, M. J. Selecting and maintaining a diverse T-cell repertoire. Nature 402, 255-262, doi:10.1038/46218 (1999).
[9] Cobbold, S. P., Martin, G., Qin, S. & Waldmann, H. Monoclonal antibodies to promote marrow engraftment and tissue graft tolerance. Nature 323, 164-166, doi:10.1038/323164a0 (1986).
[10] Pilat, N. & Wekerle, T. Transplantation tolerance through mixed chimerism. Nat Rev Nephrol 6, 594-605, doi:10.1038/nrneph.2010.110 (2010).
[11] Kawai, T. et al. HLA-mismatched renal transplantation without maintenance immunosuppression. N Engl J Med 358, 353-361, doi:10.1056/NEJMoa071074 (2008).
[12] Blazar, B. R., Murphy, W. J. & Abedi, M. Advances in graft-versus-host disease biology and therapy. Nat Rev Immunol 12, 443-458, doi:10.1038/nri3212 (2012).

Although specific embodiments of the present disclosure have been described above, it should be understood by those skilled in the art that these are merely illustrative, and various changes or modifications may be made to these embodiments without departing from the principles and essence of the present disclosure. Therefore, the scope of protection of the present disclosure is defined by the appended claims.

## Claims

1. A method for reducing or eliminating allograft rejection, comprising the following steps:
expressing donor-derived MHC and/or transplanting donor-derived thymic epithelial cells in the thymic tissue of a recipient using a thymus vaccine; the thymus vaccine comprises a thymic gene vaccine and/or a thymocyte vaccine;
preferably, the method is performed for non-therapeutic purposes; and/or, the method is performed *in vitro* or *ex vivo*.

2. The method according to claim 1, wherein the expression of donor-derived MHC is achieved by injecting the thymic gene vaccine into the thymic tissue of the recipient;
preferably, the thymic gene vaccine comprises:
(1) a gene expression vector;
(2) polynucleotides encoding donor-derived MHC class I molecules and MHC class II molecules, respectively;
wherein the polynucleotides are operably linked to the gene expression vector, and the MHC background of the donor is different from that of the recipient;
more preferably, the gene expression vector comprises a plasmid vector, a phage vector, a non-viral vector, and a viral vector; and/or, the polynucleotides encoding donor-derived MHC class I molecules and MHC class II molecules are contained within a single gene expression vector or within separate gene expression vectors.

3. The method according to claim 2, wherein the viral vector is a lentiviral vector or an adeno-associated viral (AAV) vector, such as AAV2/8;
preferably, the thymic gene vaccine comprises AAV-CMV-H2-Kb α chain-IRES-H2-Db α chain and AAV-CMV-I-Ab α chain-IRES-β chain;
more preferably, the thymic gene vaccine comprises isolated nucleic acids having the nucleotide sequences as set forth in SEQ ID NO: 1 and SEQ ID NO: 2, respectively; and/or, the thymic gene vaccine is injected into the left and right lobes of the thymus of the recipient, at 1 to 5 sites in each lobe, such as 3 sites.

4. The method according to claim 1, wherein the thymocyte vaccine is prepared by the following steps: isolating thymic epithelial cells using an anti-Epcam antibody to obtain the thymocyte vaccine;
preferably, the thymocyte vaccine is transplanted into the left and right lobes of the thymus of the recipient, at 1 to 3 sites in each lobe; and/or, the thymocyte vaccine is administered at a dose of 2 × 10⁶ thymic epithelial cells per recipient;
more preferably, the thymic epithelial cells are suspended in phosphate-buffered saline (PBS) to prepare the thymocyte vaccine.

5. The method according to claim 2 and/or claim 4, wherein following injection of the thymus vaccine, donor-reactive T cells are eliminated from newly matured T cells;
preferably, prior to injection of the thymus vaccine, the method further comprises the following steps:
(i) injecting or not injecting IFN-γ;
(ii) injecting a T cell depleting agent or an immunosuppressant; for example, the T cell depleting agent is antibodies 2.43 and GK1.5;
(iii) irradiation; for example, irradiation at 1.5 to 3 Gy for 1 to 2 minutes on the second day.

6. A method for tissue or organ transplantation, comprising the following steps:
A. reducing or eliminating allograft rejection using the method according to any one of claims 1 to 5;
B. performing cell, tissue, or organ transplantation before or after peripheral blood T cells of the recipient has returned to normal levels;
preferably, the tissue or organ is selected from skin, islet, nerve, kidney, liver, heart, small intestine, fat, and bone, any one or any combination thereof.

7. A thymic gene vaccine for expressing donor-derived major histocompatibility complex (MHC) antigens in the thymus of a recipient, comprising:
(1) a gene expression vector;
(2) polynucleotides encoding donor-derived MHC class I molecules and MHC class II molecules, respectively;
wherein the polynucleotides are operably linked to the gene expression vector, and the MHC background of the donor is different from that of the recipient;
preferably, the gene expression vector comprises a plasmid vector and a viral vector; and/or, the polynucleotides encoding donor-derived MHC class I molecules and MHC class II molecules are contained within a single gene expression vector or within two separate gene expression vectors.

8. The thymic gene vaccine according to claim 7, wherein the viral vector is a lentiviral vector or an adeno-associated viral (AAV) vector, such as AAV2/8;
preferably, the thymic gene vaccine comprises AAV-CMV-H2-Kb α chain-IRES-H2-Db α chain and AAV-CMV-I-Ab α chain-IRES-β chain;
more preferably, the thymic gene vaccine comprises isolated nucleic acids having the nucleotide sequences as set forth in SEQ ID NO: 1 and SEQ ID NO: 2, respectively.

9. A thymocyte vaccine prepared by the following steps: isolating thymic epithelial cells using an anti-Epcam antibody to obtain the thymocyte vaccine;
preferably, the thymic epithelial cells are administered at a dose of 2 × 10⁶ cells per recipient; and/or, the thymic epithelial cells are suspended in phosphate-buffered saline (PBS) to prepare the thymocyte vaccine.

10. A thymocyte vaccine kit, comprising the thymic gene vaccine according to claim 7 or 8, or the thymocyte vaccine according to claim 9;
optionally further comprising: (i) IFN-γ; and/or
(ii) a T cell depleting agent or an immunosuppressant.

11. Use of the thymic gene vaccine according to claim 7 or 8, the thymocyte vaccine according to claim 9, or the thymocyte vaccine kit according to claim 10 in the manufacture of an immune preparation for reducing or eliminating allograft rejection, or in reducing or eliminating allograft rejection.
